# EUROPEAN PATENT APPLICATION

(11) **EP 1 570 859 A2**
(43) Date of publication of application: **07.09.2005**
(21) Application number: 05012628.3
(22) Date of filing: 22.03.2001
(51) Int. Cl.: A61K 41/00, A61P 27/02

(54) **Improved treatment of neovascularization**

(30) Priority: 24.03.2000 US 191807 P
(62) Divisional of application: 01923695.9
(71) Applicant: Novartis AG, 4056 Basel (CH); Novartis Pharma GmbH, 1230 Wien (AT)
(72) Inventor: Brazzell, Romulus Kimbro, Alpharetta, GA 30201 (US)
(74) Representative: von Sprecher, Georg

(57) **Abstract**

The present invention describes an improved photodynamic treatment to treat subfoveal choroidal neovascularization (CNV).

## Description

The invention relates to an improved method to treat subfoveal choroidal neovascularization (CNV) by use of an anti-angiogenic agent as an adjunct to photodynamic therapy (PDT) also called photodynamic treatment.

The present treatment of age related macular degeneration (AMD) with photodynamic therapy using an appropriate photosensitizer leads to excellent short-term results for treating CNV and is a significant improvement over laser photocoagulation. However, it has been demonstrated that in patients treated with PDT there is a recurrence of choroidal neovascularization within the treatment area and/or development of new lesions outside the original lesions (so called progression) such that repeated PDT is required. Therefore a pharmaceutical treatment which could be used in conduction with PDT, and which prevents the growth of new vessels would be a significant advancement and would be advantageous for the treatment of CNV. The prevention of new unwanted neovasculature could reduce the number of PDT treatments required in some subjects. The present techniques may also be useful for treating other types of ocular tissue as well, such as retinal neovascular lesions.

Accordingly, the present invention describes in a first aspect a method for an improved treatment of unwanted neovasculature due to CNV in a subject, which method comprises:
(a) administration of an effective amount of an anti-angiogenic drug to said subject;
(b) administration of an effective amount of a photosensitive agent to said subject; and
(c) irradiating said unwanted neovasculature with light having a wavelength absorbable said photosensitive agent.

The present invention relates in a further aspect to the use of an anti-angiogenic drug in conjunction with a photosensitive agent in the preparation of a medicament for the improved photodynamic treatment of unwanted neovasculature due to CNV in a subject, preferably a human subject.

The invention relates in a further aspect to the use of an anti-angiogenic drug in conjunction with a photosensitive agent in the preparation of a medicament for the improved photodynamic treatment of unwanted neovasculature due to CNV in a subject, preferably human subject, wherein said improved photodynamic treatment comprises the steps of:
(a) administration of an effective amount of an anti-angiogenic drug to said subject;
(b) administration of an effective amount of a photosensitive agent to said subject; and
(c) irradiating said unwanted neovasculature with light having a wavelength absorbable said photosensitive agent.

It has now been found that administration of an anti-angiogenic can be used in conjunction with PDT for the treatment of a subject having unwanted ocular neovasculature as a result of CNV.

PDT as a treatment is well known in the art, and generally involves the use of a photosensitize agent activated by a laser. A preferred PDT treatment having a photosensitize agent and laser treatment protocol is disclosed in the issued European patent EP 680'365 B1 and in the International application WO 97/33619. In PDT, the photosensitive agent lodges in the ocular tissue affected by CNV (i.e., the target occular tissue) and is activated by a laser having a wavelength absorbable by the photosensitive agent. In the present invention, the anti-angiogenic drug is administered before, after and / or simultaneously with the photosensitizer used in the PDT treatment. The combination of PDT and anti-angiogenic drug is referred to as adjunctive PDT.

The anti-angiogenic may be administered either sequentially or simultaneously with the photosensitive agent, the preferred method being sequential. Therefore, the term "in conjunction with" shall be construed in accordance to the definitions as provided within this disclosure. As an example of sequential treatment, an anti-angiogenic drug may be administered for 1 to 4 weeks, more preferably 0.5 to 1.5 weeks before administration of the PDT photosensitizer. In an alternative sequential treatment, the anti-angiogenic may be administered 0 to 4 weeks, more preferably 0 to 1 weeks after administration of the PDT photosensitie agent. If necessary, the anti-angiogenic may be sequentially administered both before and after PDT according to the schedule described above. Alternatively, the treatment is considered simultaneous if the anti-angiogenic is co-administered with the photosensitizer. Particular subjects may require multiple adjunctive PDT treatments or adjunctive PDT treatments with the anti-angiogenic and particular adjunctive PDT treatments may require multiple administrations of the anti-angiogenic drug.

Anti-angiogenic drugs, as the term is used herein mean drugs that work by preventing, inhibiting or reversing the growth of new blood vessels via the process commonly known as angiogenesis. Examples of anti-angiogenic drugs useful in adjunctive PDT include staurosporins, for example N-benzoyl-staurosporine, somatostatins, such as octreotide 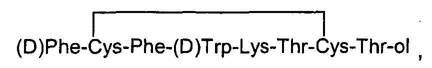 and steroids, such as triamcinolone. Other anti-angiogenic drugs useful in the present invention are VEGF inhibitors, such as CGP 79987D, CGP 57 148B or CGP 53 716, 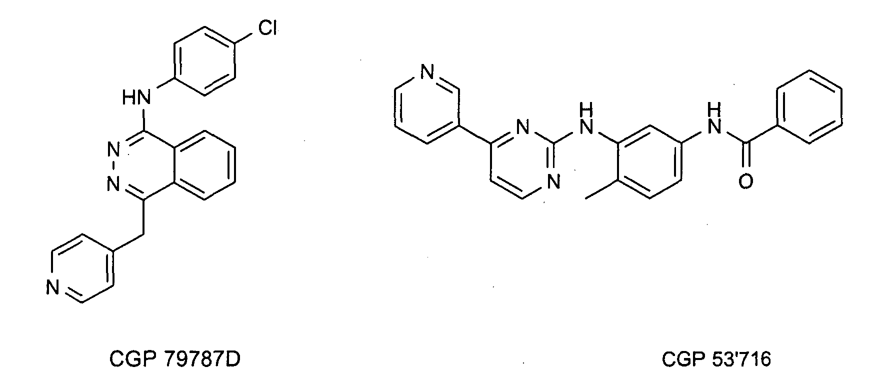 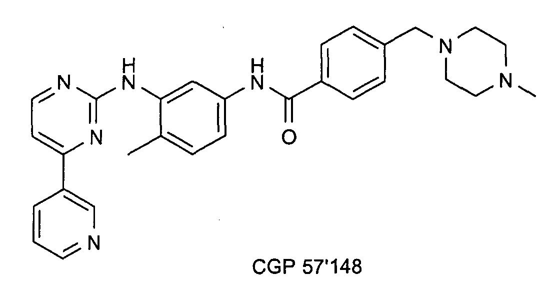 and the like. These anti-angiogenic drugs are particularly useful to inhibit the recurrence, reopening, development and / or progression of blood vessel growth that occurs during choroidal neovascularization, and offer significant benefits in adjunctive PDT.

A preferred anti-angiogenic drug is selected from inhibitors of protein kinase C (PKC) (e.g., N-benzoyl-staurosporine), inhibitors of growth hormone and IGF-1 (e.g., octreotide), inhibitors of vascular endothelial growth factor (VEGF) (e.g., CGP 79787, N-benzoyl-staurosporine, CAM 781), inhibitors of cyclooxgenase II (e.g., diclofenac, COX 189), inhibitors of angiotensin II (e.g., valsartan), inhibitors of NF-kappa B, and PLA2 antagonists, more preferably from PKC inhibitors, VEGF inhibitors and from inhibitors of growth hormone and IGF-1.

A highly preferred anti-angiogenic drug is selected from an inhibitor of PKC and VEGF, in particular from an inhibitor of PKC. A highly preferred anti-angiogenic drug is selected from N-benzoyl-staurosporine, CGP 79787 and octreotide and in particular from N-benzoyl-staurosporine.

The preferred photosensitizers are selected from the group of a chlorine, a bacteriochlorine, a phthalocyanine, a porphyrin, a purpurin, a merocyanine, a pheophorbide and a psoralen.

A highly preferred photosensitizer is selected from the porphyrins and is typically the so-called green porphyrin or BPD-MA.

Any of the photosensitive compounds described above can be used in the method of the invention. Of course, mixtures of two or more photosensitive compounds can also be used; however, the effectiveness of the treatment depends on the absorption of light by the photosensitive compound so that if mixtures are used, components with similar absorption maxima are preferred.

The nature of the formulation used to deliver the anti-angiogenic drug or photosensitive agent will depend in part on the mode of administration and on the nature of the anti-angiogenic drug and the photoactive agent selected. Any pharmaceutically acceptable excipient, or combination thereof, appropriate to the particular active compounds may be used. Thus, the photosensitive agents or anti-angiogenic compounds may be administered as an aqueous composition, as a transmucosal or transdermal composition, as a subtenons or intraocuclar injection or in an oral formulation. The formulation may also include liposomes. Liposomal compositions are particularly preferred especially where the photoactive agent is a green porphyrin. The anti-angiogenic drug is preferably administered via an aqueous carrier.

The above mentioned compounds can be administered in any of a wide variety of ways, for example, orally, parenterally, or rectally, or the compound may be placed directly in or on the eye. Parenteral administration, such as intravenous, intramuscular, or subcutaneous, is preferred for the photosensitizer. Intravenous injection is especially preferred. Oral administration or ocular administration is preferred for administration of the anti-angiogenic agent.

The dose of the above compounds can vary widely depending upon the mode of administration; the formulation in which it is carried, such as in the form of liposomes, or whether it is coupled to a target-specific ligand, such as an antibody or an immunologically active fragment. As is generally recognised, there is a nexus between the type of photoactive agent, the formulation, the mode of administration, and the dosage level. The anti-antigenic drug is administered in a manner and amount sufficient to effect drug interaction with the unwanted neovasculature. The photosensitive agent is administered in an amount effective to provide closure to the unwanted neovasculature.

While various photoactive compounds require different dosage ranges, if green porphyrins are used, a typical dosage is of the range of 0.1-50 mg/m² of body surface area, preferably from about 1-10 mg/m² and even more preferably about 2-8 mg/m².

While various anti-angiogenic compounds require different dosage ranges, a typical dosage is of the range of 1-500 mg/kg (of body weight) preferably from about 10-250 mg.

The irradiation (laser power, irradiation duration) is carried out in accordance to the prior art mentioned above, for example in accordance to the light treatment protocol of the disclosure of WO 97/33619.

## Claims

1. Use of an anti-angiogenic drug in conjunction with a photosensitive agent in the preparation of a medicament for the improved photodynamic treatment of unwanted neovasculature due to CNV (subfoveal choroidal neovascularization) in a subject, wherein said anti-angiogenic drug is selected from PKC (protein kinase C) inhibitors, VEGF (vascular endothelial growth factor) inhibitors, from inhibitors of growth hormone and inhibitors of the IGF-1 (insulin like growth factors).

2. Use of claim 1, wherein said anti-angiogenic drug is selected from PKC (protein kinase C) inhibitors, and VEGF (vascular endothelial growth factor) inhibitors.

3. Use of claim 1, wherein said anti-angiogenic drug is selected from CGP 79987D, CGP 57 148B and CGP 53 716,

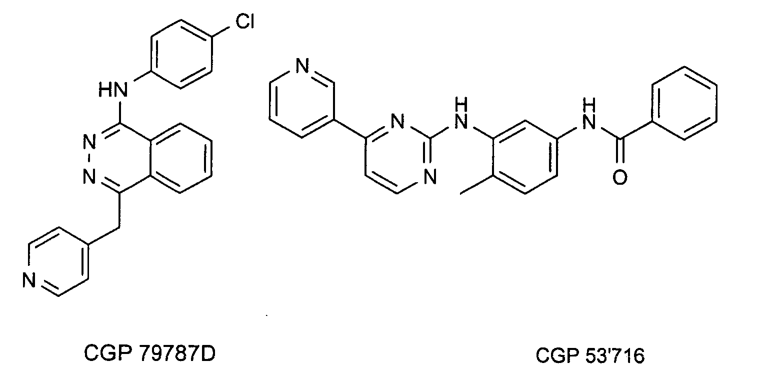

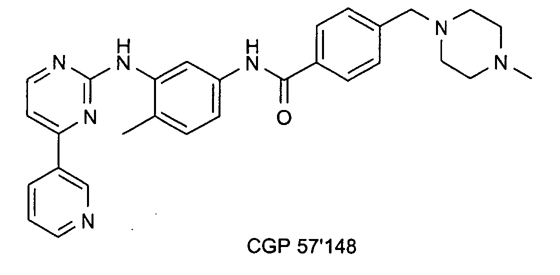

4. Use of claim 3, wherein said anti-angiogenic drug is CGP 79787D.

5. Use of claim 1, wherein said photosensitizer agent is selected from a porphyrin and a purpurin and more preferably from a porphyrin.

6. Use of claim 1, wherein said photosensitizer agent is green porphyrin or BPD-MA .

7. Use of an anti-angiogenic drug as adjunct to photodynamic therapy (PDT) in the manufacture of a medicament in the treatment of the progression of choroidal neovascularization, wherein said anti-angiogenic drug is selected from N-benzoyl-staurosporine, CGP 79787D and octreotide, in particular wherein said anti-angiogenic drug is CGP 79787D.
